(19) Europäisches Patentamt
European Patent Office
Office européen des brevets

(11) **EP 1 050 270 B1**

(12) **FASCICULE DE BREVET EUROPEEN**

(45) Date de publication et mention
de la délivrance du brevet:
**03.12.2008 Bulletin 2008/49**

(51) Int Cl.:
***A61B 5/0428*** (2006.01)

(21) Numéro de dépôt: **00440127.9**

(22) Date de dépôt: **05.05.2000**

(54) **Procédé et dispositif pour l'acquisition d'un électrocardiogramme**

Verfahren und Vorrichtung zur Erfassung von Elektrokardiogrammen

Method and device for acquisition of electrocardiograms

(84) Etats contractants désignés:
**AT BE CH CY DE DK ES FI FR GB GR IE IT LI LU MC NL PT SE**

(30) Priorité: **07.05.1999 FR 9905963**

(43) Date de publication de la demande:
**08.11.2000 Bulletin 2000/45**

(73) Titulaire: **Schiller Medical**
**67160 Wissembourg (FR)**

(72) Inventeur: **Fischer, Roland**
**67160 Wissembourg (FR)**

(74) Mandataire: **Metz, Paul**
**Cabinet METZ PATNI**
**B.P. 63**
**67024 Strasbourg Cedex 01 (FR)**

(56) Documents cités:
**EP-A- 0 247 991**      **FR-A- 2 685 968**

**Description**

La présente invention concerne le domaine de l'acquisition et du traitement de signaux physiologiques, notamment en vue d'améliorer le rapport signal/bruit, et a pour objet un procédé et un dispositif d'acquisition d'un signal d'électrocardiogramme (ECG), sensiblement dépourvu de bruit, en particulier de bruit provenant de variations du champ magnétique local.

L'acquisition de signaux physiologiques pose souvent des problèmes de récupération fidèle du signal, puisque généralement faible et noyé dans un bruit important.

La difficulté est encore accrue lorsque l'acquisition est réalisée dans un environnement RMN et que le signal concerné est un électrocardiogramme (ECG).

Or, l'électrocardiogramme est le signal physiologique par excellence pour surveiller l'état d'un patient, mais également en tant que signal de séquencement, notamment pour un imageur RMN, par exemple en tant que signal de déclenchement d'une séquence d'acquisition (plus connue sous la désignation "triggering") et/ou en tant que signal de déclenchement d'une fenêtre d'acquisition (plus connue sous la désignation "gating").

En effet, ce signal peut être exploité de différentes manières, notamment par visualisation, analyse de formes, comptage ou analogue, en vue de déterminer des caractéristiques morphologiques, de fréquence ou autre.

Le signal d'électrocardiogramme est un signal répétitif dont chaque séquence est constituée par la juxtaposition de plusieurs ondes (P, QRS, T, ST).

Le débruitage du signal est bien entendu important en termes de monitorage. En effet, pour que le médecin puisse suivre en temps réel l'évolution du patient, il faut qu'il dispose d'un accès clair à l'information et un ECG bruité ne s'avère être d'aucune utilité.

La détection des pics dans le complexe QRS est d'une importance capitale. D'une part, pour déterminer la fréquence cardiaque, mais également pour pouvoir synchroniser la prise d'images d'un imageur RMN sur l'ECG ("triggering"/"gating"). Cette synchronisation cardiaque permet d'exciter chaque coupe à un moment qui est toujours le même dans le cycle cardiaque, et donc d'avoir une image de cette coupe qui s'affranchit des phénomènes de mouvements.

Actuellement, cette phase de filtrage du signal d'électrocardiogramme est généralement réalisée par des circuits analogiques ou des cartes dédiées dont les caractéristiques sont figées et les performances limitées et qui ne parviennent pas à débarrasser efficacement le signal d'ECG de ses artefacts et parasites dûs aux perturbations électromagnétiques prévalant en particulier au cours des examens RMN.

Ces perturbations électromagnétiques proviennent essentiellement des pics et changements locaux du champ magnétique principal Bo résultant de l'action des gradients mis en oeuvre au cours des investigations RMN. Elles introduisent un signal de bruit dans la boucle de mesure du signal d'ECG, dont l'intensité peut être équivalente, voire supérieure, à celle du signal d'ECG.

On notera que Bo est orienté selon l'axe longitudinal du patient, dans le cas d'un appareil RMN à tunnel, et perpendiculairement au plan coronal du patient dans le cas d'un appareil RMN à entrefer du type "ouvert".

En considérant que l'orientation du champ principal Bo correspond à l'axe Z d'un repère spatial orthogonal (X, Y, Z), les gradients linéaires en X, Y et Z peuvent être définis comme suit :

$$grad.X = \frac{d\,Bz}{dx}, \quad grad.Y = \frac{d\,Bz}{dy} \quad et\ grad.Z = \frac{d\,Bz}{dz}$$

Pour tenter de supprimer ce signal de bruit induit, plusieurs solutions ont déjà été mises en oeuvre.

Ainsi, il a été proposé de soumettre le signal d'ECG à un filtre sensible à la vitesse d'accroissement de la tension du signal réglé à une valeur légèrement supérieure à la valeur maximale typique du dV/dt de l'ECG.

Toutefois, ce procédé ne permet pas de supprimer le bruit induit par les séquences d'application et de suppression de différents gradients de fréquence élevée.

Il a également été proposé de dériver le signal d'ECG prélevé en deux signaux secondaires, de retarder l'un de ces derniers d'une durée correspondant à un multiple de la période du signal de l'ECG et d'en extraire la composante de bruit, et ensuite d'opérer une soustraction entre le signal non retardé et la composante de bruit d'une période précédente.

Néanmoins, ce procédé ne permet d'aboutir à une amélioration notable du signal d'ECG que si le bruit induit, et donc les conditions électromagnétiques, sont sensiblement identiques sur plusieurs périodes du signal d'ECG et si ce dernier est relativement régulier. Or, dans la pratique, ces cas ne sont pas fréquents et il peut en résulter une déformation notable du signal d'ECG.

La présente invention a notamment pour but de pallier les inconvénients précités.

Le brevet français FR 2 685 968 concerne un dispositif de transmission vers un moniteur de signaux physiologiques d'un patient soumis à un examen d'imagerie par résonnance magnétique nucléaire.

Selon ce dispositif, un module de traitement et de conversion des signaux disposé près du patient est relié à des capteurs placés sur le patient et un module de réception et de reconversion des signaux reçoit ceux-ci transmis sous forme optique par une fibre optique et les traite pour permettre leur exploitation dans un moniteur.

Selon cette invention, on s'affranchit des parasites venant du milieu ambiant et des perturbations apportées par celui-ci en convertissant les signaux électriques en signaux optiques.

La présente invention permet d'éviter d'une autre façon la perturbation du signal par des parasites du milieu électromagnétique perturbateur dans lequel baigne le patient.

Il consiste à relever simultanément sur le patient dans des conditions de perturbation sensiblement identiques un second signal de mesure renfermant au moins le bruit et d'exploiter un signal différentiel affranchi du bruit. La transmission s'effectue en modelant le signal en fréquence et en utilisant la conversion opto-électrique.

La demande européenne EP 0 247 991 se rapporte à une méthode et à un dispositif multicanaux permettant de capter et de transmettre des signaux lents de faible amplitude. Il s'agit notamment de la transmission de la différence d'amplitude des échantillons entre deux interrogations consécutives.

La transmission selon la présente invention ne procède pas de cette façon.

On transmet en temps réel un signal différentiel entre un premier signal de mesure contenant le bruit perturbateur et un second signal de mesure renfermant le bruit résultant d'une mesure effectuée dans des conditions de perturbation sensiblement identiques.

La présente invention se distingue de ces deux dépôts antérieurs par deux mesures dans des endroits sensiblement identiquement perturbés et l'exploitation d'un signal différentiel sensiblement exempt de parasites perturbateurs.

A cet effet, elle a pour objet un procédé d'acquisition d'un électrocardiogramme (ECG) d'un patient situé dans un environnement électromagnétique perturbateur, consistant à relever sur le patient un signal d'ECG additionné du bruit au niveau de la région cardiaque en tant que signal différentiel résultant des signaux délivrés par deux électrodes faisant partie d'une première boucle de mesure, consistant, en outre, à relever simultanément sur le patient, dans des conditions de perturbations sensiblement identiques, un second signal de mesure renfermant au moins le bruit, en tant que signal différentiel résultant des signaux délivrés par deux électrodes faisant partie d'une seconde boucle de mesure distincte de la première, puis à additionner au ou à soustraire du premier signal d'ECG bruité, en temps réel, ledit second signal de mesure, en fonction de la polarité du bruit dans ce dernier par rapport au bruit dans le signal d'ECG bruité et, enfin, à traiter, à convertir, à transmettre et/ou à afficher en temps réel le signal résultant, représentatif de l'ECG et sensiblement dépourvu de bruit.

L'invention a également pour but de transmettre le signal EGC dépourvu de bruit par une liaison de transmission sans récupération de parasites ou de signaux étrangers perturbateurs.

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels :

- la figure 1 est une vue en perspective d'une tranche transversale d'un patient, à hauteur du coeur, illustrant l'implantation des électrodes selon une première variante de réalisation du procédé selon l'invention, en particulier par rapport au champ principal Bo dans un appareil RMN à tunnel ;
- la figure 2 est une vue schématique de dessus montrant la disposition des électrodes représentées à la figure 1 ;
- la figure 3 est une vue schématique de dessus illustrant une autre disposition des électrodes selon une deuxième variante de réalisation de l'invention ;
- la figure 4 est une vue schématique de dessus illustrant une autre disposition des électrodes selon une troisième variante de réalisation de l'invention ;
- la figure 5 est un schéma-bloc illustrant les différentes composantes d'un appareil d'acquisition de l'ECG selon un mode de réalisation particulier de l'invention ;
- les figures 6A et 6B sont des diagrammes temporels de signaux d'électrocardiogrammes recueillis durant l'application d'une séquence RMN, respectivement sans et avec mise en oeuvre du procédé selon l'invention, et,
- les figures 7A et 7B sont également des diagrammes temporels de signaux d'électrocardiogrammes recueillis avant et durant l'application d'une séquence RMN, respectivement sans et avec mise en oeuvre du procédé selon l'invention.

Ainsi, le procédé d'acquisition de l'électrocardiogramme (ECG) d'un patient 1 situé dans un environnement électromagnétique perturbateur, consiste tout d'abord à relever sur ledit patient 1 un signal d'ECG additionné du bruit S1 au niveau de la région cardiaque en tant que signal différentiel résultant des signaux délivrés par deux électrodes 3, 3' faisant partie d'une première boucle de mesure 2.

Conformément à l'invention, ledit procédé consiste, en outre, à relever simultanément sur le patient 1, dans des conditions de perturbations sensiblement identiques, un second signal de mesure S2 renfermant au moins le bruit, en tant que signal différentiel résultant des signaux délivrés par deux électrodes 5, 5' faisant partie d'une seconde boucle de mesure 4 distincte de la première 2, puis à additionner au ou à soustraire du premier signal d'ECG bruité S1, en temps réel, ledit second signal de mesure S2, en fonction de la polarité du bruit dans ce dernier par rapport au bruit dans le signal d'ECG bruité Si et, enfin, à traiter, à convertir, à transmettre et/ou à afficher en temps réel le Signal Résultant SR, représentatif de l'ECG et sensiblement dépourvu de bruit.

Pour s'assurer de la présence dans le signal résultant SR d'au moins une composante représentative de l'ECG, il est prévu que la seconde boucle de mesure 4 soit constituée et disposée de telle manière, qu'en cas d'acquisition par elle d'une composante de signal représentative de l'ECG en plus du ou mélangée au signal de bruit, ladite composante de signal représentative de l'ECG présente une polarité opposée et/ou une amplitude inférieure à celle(s) de la composante de signal représentative de l'ECG recueillie par la première boucle de mesure 2.

En s'inspirant notamment du mode de création et d'application des gradients magnétiques au cours des expériences RMN et des résultats de leurs actions cumulées en terme de création et de variation des intensités des champs magnétiques locaux, l'inventeur a pu déterminer les mesures à prendre pour optimiser la réduction de la composante de signal relative au bruit induit par lesdits gradients dans le signal d'ECG résultant SR.

Ainsi, dans le cas d'un patient 1 placé dans un Imageur à Résonance Magnétique Nucléaire ou un appareil d'analyse RMN analogue, les dimensions apparentes pour le flux magnétique, dans un plan PP perpendiculaire à la direction du champ principal Bo, des première et seconde boucles de mesure 2 et 4 formées par les fils de liaison 2' et 4' des électrodes respectives 3, 3' et 5, 5' et les tissus 2", 4" s'étendant entre les deux électrodes 3, 3' ; 5, 5' d'une même boucle de mesure 2 ; 4 sont déterminées pour être similaires, voire identiques.

Dans ce contexte d'application, le signal résultant SR pourra être avantageusement utilisé pour la surveillance par monitorage du patient et pour le déclenchement de l'IRMN ("triggering" et/ou "gating").

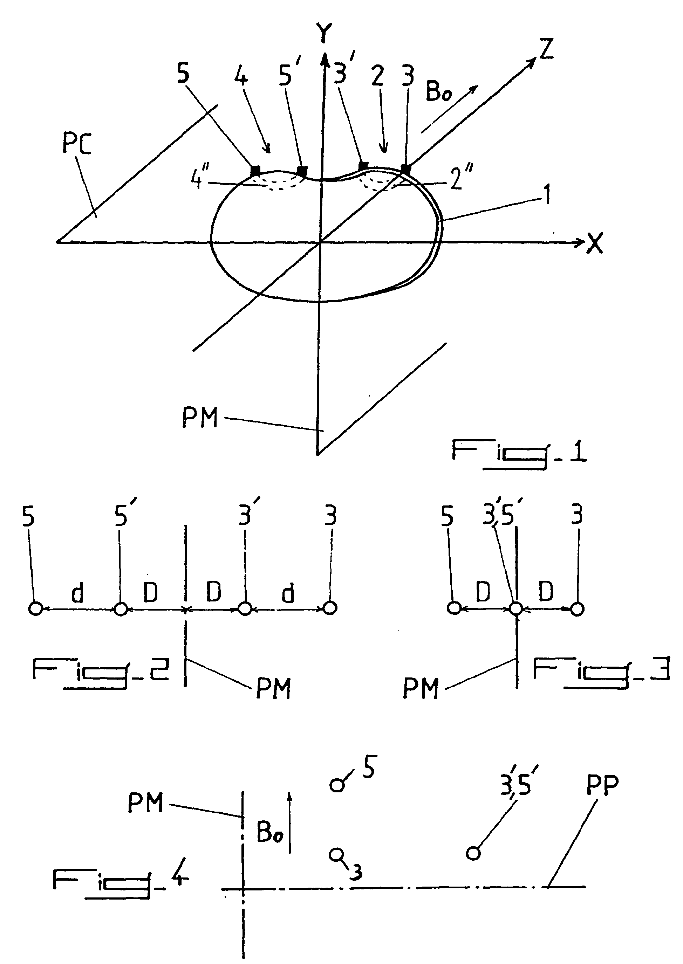

Comme il ressort d'une comparaison entre les figures 1 et 2 des dessins annexés, d'une part, et les figures 3 et 4, d'autre part, les deux boucles de mesure 2 et 4 pourront, en fonction des applications et de la constitution des moyens capteurs utilisés, soit être physiquement séparées et indépendantes, soit comporter une électrode commune 3', 5'.

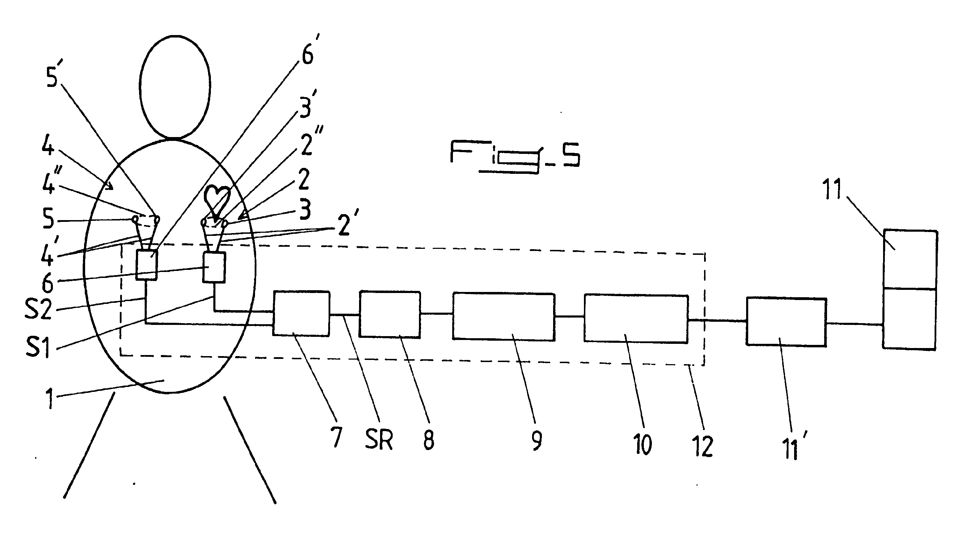

Conformément à un premier mode de réalisation de l'invention, les première et seconde boucles de mesure 2 et 4 sont disposées sensiblement symétriquement par rapport au plan médian PM du patient 1 perpendiculaire au plan coronal PC de ce dernier, les électrodes 3, 3' ; 5, 5' des deux boucles de mesure 2 et 4 étant préférentiellement mais non obligatoirement disposées de manière alignée et en présentant un espacement similaire entre les électrodes de chaque paire 3 et 3'; 5 et 5' (voir figures 1 à 3 et 5 des dessins annexés).

En effet, l'inventeur a pu constater que les signaux de bruit relevés par des boucles de mesure 2 et 4 configurées et disposées tel que décrit ci-dessus étaient sensiblement identiques dans le cadre des expériences RMN courantes faisant appel à des gradients de champ magnétique.

Selon un second mode de réalisation de l'invention représenté sur la figure 4, les trois électrodes 3, 3' ; 5, 5' formant les deux boucles de mesure 2 et 4 sont disposées dans une configuration triangulaire au niveau de la région cardiaque de telle manière que la seconde boucle de mesure 4 relève une composante de signal représentative de l'ECG de polarité opposée et d'amplitude sensiblement égale à celle de la composante de signal représentative de l'ECG relevée par la première boucle de mesure 2.

Enfin, en vue de son exploitation en temps réel, notamment dans le cadre d'une surveillance de l'état d'un patient soumis à un examen RMN, le signal SR résultant de l'addition ou de la soustraction des signaux différentiels S1 et S2 délivrés par les deux boucles de mesure 2 et 4 peut être traité par un filtre passe-bas 8, puis utilisé pour la modulation en fréquence d'une porteuse et, enfin, transmis vers un appareil 11 muni de moyens d'affichage du signal d'ECG SR filtré, le cas échéant après conversion en signal optique.

Il peut être prévu que le signal d'ECG soit éventuellement soumis à un filtrage numérique par un filtre à réponse impulsionnelle infinie du type elliptique ou dit de Cauer, par exemple du quatrième ordre.

La présente invention a également pour objet un dispositif capteur pour l'acquisition en temps réel du signal d'ECG d'un patient 1, en particulier soumis à un examen RMN, pouvant être relié à un appareil d'affichage 11 et permettant notamment la mise en oeuvre du procédé tel que décrit ci-dessus.

Ce dispositif est principalement constitué, d'une part, par deux modules 6, 6' de prélèvement de signaux physiologiques S1 et S2 fournis par deux boucles de mesure distinctes 2 et 4 comprenant chacune une paire d'électrodes 3, 3' ; 5, 5' placées sur le patient 1, au moins l'un des deux signaux physiologiques S1, S2 comprenant une composante de signal représentative de l'ECG, d'autre part, par un module 7 sommateur ou différenciateur des signaux S1 et S2 délivrés par les deux modules de prélèvement 6, 6' et, enfin, par des modules 8, 9, 10 de traitement, notamment de filtrage et de conversion, du signal SR fourni par ledit module 7 sommateur ou différenciateur.

Les modules de prélèvement 6, 6' consistent avantageusement, chacun, en un amplificateur différentiel d'instrumentation dont les entrées sont reliées aux électrodes respectives.

Les modules de traitement 8, 9, 10 pourront par exemple consister respectivement en un filtre passe-bas 8, en une unité de modulation de fréquence 9 et en un module 10 de conversion électro-optique relié à une fibre optique dont l'autre extrémité est reliée à un module 11' de conversion opto-électronique relié ou faisant partie de l'appareil 11 muni des moyens d'affichage du signal d'ECG.

De manière avantageuse, tous les modules 6, 6', 7, 8, 9, 10 sont regroupés dans un boîtier blindé 12 formant cage de Faraday, qui est relié aux électrodes 3, 3' ; 5, 5' par des fils de liaison résistifs flexibles 2", 4" et qui n'est pas en contact direct avec la peau du patient 1, les deux modules de prélèvement 6, 6' pouvant éventuellement comporter une électrode commune 3', 5'.

En outre, les différents éléments et parties formant le dispositif selon l'invention pourront être essentiellement constitués à partir de matériaux amagnétiques, rendant son utilisation parfaitement compatible avec un environnement magnétique sensible et perturbateur.

Le dispositif pour la mise en oeuvre du procédé d'acquisition décrit ci-dessus pourra notamment consister en un dispositif capteur du type décrit dans le brevet français n° 2 704 131 ou dans le brevet français n° 2 729 071.

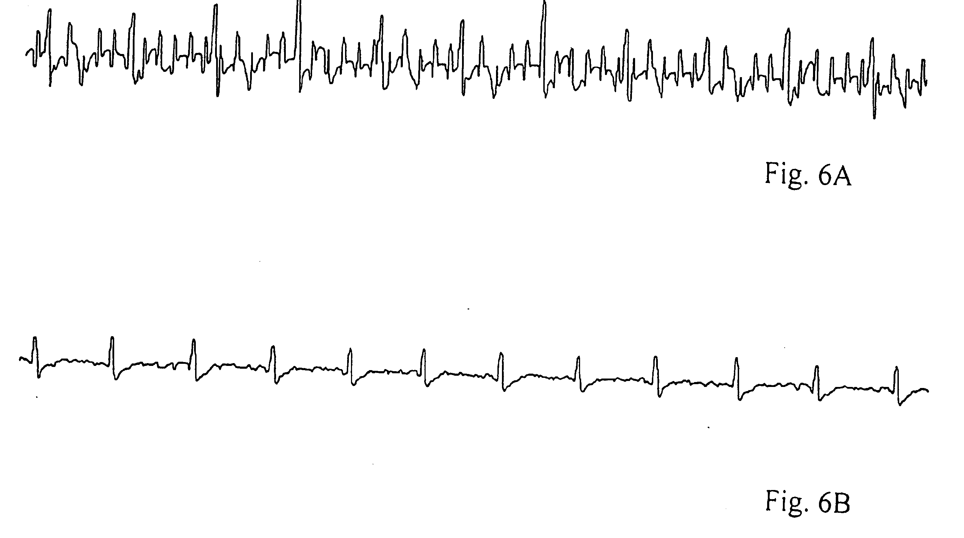

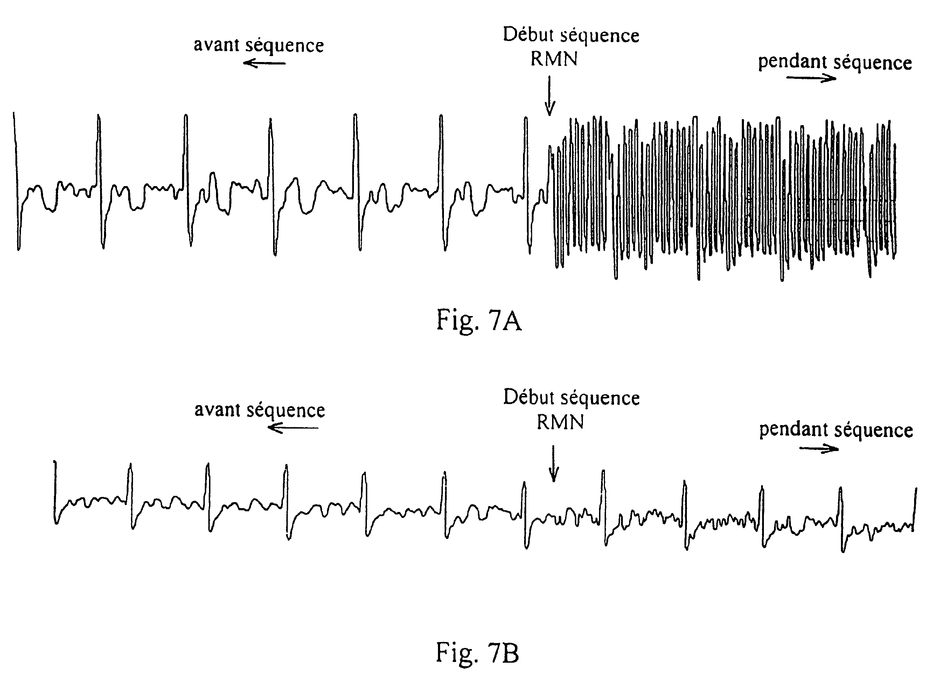

Les avantages procurés par l'invention, en terme d'amélioration de la qualité du signal d'ECG final, apparaissent très clairement à l'étude des figures 6A, 6B, 7A et 7B des dessins annexés.

Les figures 6A et 6B, d'une part, et 7A et 7B, d'autre part, représentent des signaux d'électrocardiogrammes recueillis sur un même patient et dans les mêmes conditions de positionnement des électrodes et d'environnement électromagnétique.

Ainsi, ces signaux ont été recueillis avec un placement des électrodes à environ 22 cm en avant du centre magnétique, ce qui constitue le positionnement le plus défavorable (les perturbations dues à la commutation des gradients étant maximales), et avec des gradients de magnitude 16 mT/m.

Les signaux des figures 6A et 6B ont été recueillis au cours de l'application d'une séquence RMN du type connu sous la désignation "Fast Spin Echo", alors que les signaux des figures 7A et 7B ont été recueillis avant et durant l'application d'une séquence RMN du type connu sous la désignation "Echo de Gradient" avec compensation en flux.

L'amélioration de la qualité du signal résultant de l'application du procédé selon l'invention apparaît nettement en comparant, d'une part, la figure 6A avec la figure 6B et, d'autre part, la figure 7A avec la figure 7B.

L'invention reste valable même si la disposition des électrodes change.

## Revendications

**1.** Procédé d'acquisition de l'électrocardiogramme (ECG), sensiblement dépourvu de bruit, d'un patient situé dans un environnement électromagnétique perturbateur engendrant un bruit dans le signal de mesure, consistant à relever sur le patient au niveau de sa région cardiaque un premier signal de mesure (S1) qui est un signal D'ECG additionné du bruit en tant que signal recueilli par deux électrodes (3, 3') faisant partie d'une première boucle de mesure (2), et consistant, à relever en plus et simultanément sur le patient (1), par une deuxième boucle de mesure (4) distincte de la première comprenant deux électrodes (5, 5'), un second signal de mesure (S2) renfermant au moins le bruit, puis en temps réel à additionner au, ou à soustraire du, premier signal d'ECG bruité (S1), le second signal de mesure (S2) renfermant au moins le bruit, à additionner ou à soustraire en fonction de la polarité du bruit dans ce dernier signal (S2) par rapport à celle du bruit dans le signal d'ECG bruité (S1), la seconde boucle de mesure (4) étant constituée et disposée pour permettre l'acquisition par elle du signal d'ECG en plus du bruit ou mélangé au bruit, ledit signal d'ECG présentant une amplitude inférieure à celle du signal d'ECG recueilli par la première boucle de mesure (2) et enfin, à traiter, à convertir, à transmettre et/ou à afficher en temps réel le signal résultant (SR), représentatif de l'ECG et sensiblement dépourvu du bruit.

**2.** Procédé d'acquisition selon la revendication 1, **caractérisé en ce que** la seconde boucle de mesure (4) est constituée et disposée de telle manière qu'en cas d'acquisition par elle d'une composante de signal représentative de l'ECG en plus du ou mélangée au signal de bruit, ladite composante de signal représentative de l'ECG présente une polarité opposée et/ou une amplitude inférieure à celle(s) de la composante de signal représentative de l'ECG recueillie par la première boucle de mesure (2).

**3.** Procédé d'acquisition selon la revendication 1 ou 2, **caractérisé en ce que**, dans le cas d'un patient (1) situé dans un Imageur à Résonance Magnétique Nucléaire ou un appareil d'analyse RMN analogue, les dimensions apparentes pour le flux magnétique, dans un plan (PP) perpendiculaire à la direction du champ principal (Bo), des première et seconde boucles de mesure (2 et 4) formées par les fils de liaison (2' et 4') des électrodes respectives (3, 3' et 5, 5') et les tissus (2", 4") s'étendant entre les deux électrodes (3, 3'; 5, 5') d'une même boucle de mesure (2 ; 4) sont similaires, voire identiques.

**4.** Procédé d'acquisition selon l'une quelconque des revendications 1 ou 3, **caractérisé en ce que** les première et seconde boucles de mesure (2 et 4) comportent une électrode commune (3', 5').

**5.** Procédé d'acquisition selon l'une quelconque des revendications 1 à 4, **caractérisé en ce que** les première et seconde boucles de mesure (2 et 4) passant par les électrodes sont disposées sensiblement symétriquement par rapport au plan médian (PM) du patient (1) perpendiculaire au plan coronal (PC) de ce dernier.

**6.** Procédé d'acquisition selon la revendication 5, **caractérisé en ce que** les électrodes (3, 3' ; 5 5') distinctes ou avec une électrode commune des deux boucles de mesure (2 et 4) sont disposées de manière alignée.

**7.** Procédé d'acquisition selon l'une quelconque des revendications 1 à 4, **caractérisé en ce que** les trois électrodes (3, 3' ; 5, 5') formant les deux boucles de mesure (2 et 4) sont disposées dans une configuration triangulaire au niveau de la région cardiaque de telle manière que la seconde boucle de mesure (4) relève une composante de signal représentative de l'ECG de polarité opposée et d'amplitude sensiblement égale à celle de la composante de signal représentative de l'ECG relevée par la première boucle de mesure (2).

**8.** Procédé d'acquisition selon l'une quelconque des revendications 1 à 7, **caractérisé en ce que** le signal (SR) résultant de l'addition ou de la soustraction des signaux différentiels (S1 et S2) délivrés par les deux boucles de mesure (2 et 4) est traité par un filtre passe-bas (8), puis utilisé pour la modulation en fréquence d'une porteuse et, enfin, transmis vers un appareil (11) muni de moyens d'affichage du signal d'ECG (SR) filtré, le cas échéant après conversion en signal optique.

**9.** Dispositif capteur pour l'acquisition en temps réel du signal d'ECG d'un patient situé dans un environnement électromagnétique perturbateur, en particulier soumis à un examen RMN, pouvant être relié à un appareil d'affichage et permettant la mise en oeuvre du procédé *selon l'une quelconque des revendications 1 à 9*, le dispositif étant principalement constitué, d'une part, par deux modules (6, 6') de prélèvement de signaux physiologiques (S1 et S2) le premier (S1) étant un signal EGC bruité et le second (S2) étant un signal de mesure renfermant au moins le bruit, les modules étant formés par deux boucles de mesure distinctes (2 et 4) comprenant chacune une paire d'électrodes (3, 3' ; 5, 5') *adaptées à être placées* sur le patient (1), et d'autre part, par un module (7) sommateur ou différenciateur des signaux (S1 et S2) délivrés par les deux modules de prélèvement (6, 6') et, enfin, par des modules (8, 9, 10) de traitement, notamment de filtrage et de conversion, du signal résultant (SR) fourni par ledit module (7) sommateur ou différenciateur, la seconde boucle de mesure (4) étant constituée et disposée pour permettre l'acquisition par elle du signal d'ECG en plus du bruit ou mélangé à celui-ci, ledit signal d'ECG présentant une amplitude inférieure à celle du signal d'ECG recueilli par la première boucle de mesure (2) pour traiter, convertir, transmettre et/ou afficher en temps réel le signal résultant (SR), représentatif de l'ECG et sensiblement dépourvu du bruit.

**10.** Dispositif selon la revendication 9, **caractérisé en ce que** tous les modules (6, 6', 7, 8, 9, 10) sont regroupés dans un boîtier blindé (12) formant cage de Faraday, qui est relié aux électrodes (3, 3' ; 5, 5') par des fils de liaison résistifs flexibles (2", 4") et qui n'est pas en contact direct avec la peau du patient (1).

**11.** Dispositif selon l'une quelconque des revendications 9 et 10, **caractérisé en ce que** les deux modules de prélèvement (6, 6') comportent une électrode commune (3', 5').

**12.** Dispositif selon l'une quelconque des revendications 9 à 11, **caractérisé en ce que** les différents éléments et parties formant ledit dispositif sont essentiellement constitués à partir de matériaux amagnétiques, rendant son utilisation parfaitement compatible avec un environnement magnétique sensible et perturbateur.

**Claims**

**1.** A method of obtaining the electrocardiograph (ECG), which is essentially devoid of noise, of a patient located in a turbulent electromagnetic environment generating some noise into the measurement signal, consisting of recovering from the patient near the cardiac region a first measurement signal (S1) which is an ECG signal with added noise as a collected signal by two electrodes (3, 3') forming part of a first measurement loop (2), and consisting of further and simultaneously recovering from the patient (1), by a second measurement loop (4) distinct from the first one comprising two electrodes (5, 5'), a second measurement signal (S2) incorporating at least the noise, then, in real time, adding to or subtracting from the first noisy ECG signal (S1), said second measurement signal (S2) incorporating at least the noise, adding or subtracting as a function of the polarity of the noise in said second signal (S2) relative to the noise in the noisy EGG signal (S1), said second measurement loop (4) being composed and arranged in order to allow it to acquire the ECG signal in addition to or mixed with the noise signal, said ECG signal having a inferior amplitude to the ECG signal amplitude obtained by the first measurement loop (2) and, finally, processing, converting, transmitting and/or displaying in real time the resulting signal (SR) representing the EGG, which is essentially devoid of noise.

2. A method of acquisition according to claim 1 **characterized in that** the second measurement loop (4) is composed and arranged in such a way that, if it acquires a signal component representing the EGG that is in addition to or mixed with the noise signal, said signal component representing the EGG has an opposite polarity and/or an inferior amplitude to the signal component representing the ECG obtained by the first measurement loop (2).

3. A method of acquisition according to either claim 1 or claim 2 **characterized in that**, in the case where a patient (1) is located inside a nuclear magnetic resonance imager NMI or similar analysis apparatus, the apparent dimensions for magnetic flux, in a plane (PP) perpendicular to the direction of the principal field (Bo) of the first and second measurement loops (2 and 4) formed by the connecting wires (2' and 4') of the respective electrodes (3, 3'; 5, 5') and the tissues (2", 4") extending between the two electrodes (3, 3'; 5, 5') of a single measurement loop (2; 4) are similar or even identical.

4. A method of acquisition according to any one of claims 1 through 3 **characterized in that** the first and second measurement loops (2 and 4) comprise a common electrode (3', 5').

5. A method of acquisition according to any one of claims 1 through 4 **characterized in that** the first and second measurement loops (2 and 4) passing through the electrodes are arranged essentially symmetrically relative to the median plane (PM) of the patient (1) perpendicular to the patient's coronal plane (PC).

6. A method of acquisition according to claim 5 **characterized in that** the electrodes (3, 3'; 5, 5'), either separate or with a common electrode, of the two measurement loops (2 and 4) are arranged in alignment.

7. A method of acquisition according to any one of claims 1 through 4 **characterized in that** the three electrodes (3, 3'; 5, 5') forming the two measurement loops (2 and 4) are arranged in a triangular configuration near the cardiac region in such a way that that the second measurement loop (4) obtains a signal component representing the EGG which has the opposite polarity and is essentially equal in amplitude to that of the signal component representing the EGG obtained by the first measurement loop (2).

8. A method of acquisition according to any one of claims 1 through 7 **characterized in that** the signal (SR) resulting from the addition or subtraction of the differential signals (S1 and S2) delivered by the two measurement loops (2 and 4) is processed by a low-pass filter (8), then used for frequency modulation of a carrier and finally, transmitted to an apparatus (11) equipped with a means for displaying the filtered EGG signal (SR), possibly after conversion into an optical signal.

9. A sampling apparatus for obtaining the EGG signal in real time of a patient situated in a magnetically turbulent environment, particularly a patient undergoing MRI examination, which apparatus can be connected to a display device; and in particular, an apparatus using the method according to any one of claims 1 through 9, the apparatus consisting principally of first, two modules (6, 6') for acquiring physiological signals (S1 and S2) the first signal (S1) being a noisy ECG signal and the second signal (S2) being a measurement signal incorporating at least the noise, the modules being formed by two separate measurement loops (2 and 4), each comprising a pair of electrodes (3, 3'; 5, 5') arranged in order to be placed on the patient (1) ; and second, of a module (7) which totals or differentiates the signals (S1 and S2) delivered by the two acquisition modules (6, 6') and finally, by the modules (8, 9, 10) which process, i.e., filter and convert, the resulting signal (SR) delivered by said totaling or differentiating module (7), said second measurement loop (4) being composed and arranged in order to allow it to acquire the ECG signal in addition to or mixed with the noise signal, said ECG signal having a inferior amplitude to the ECG signal amplitude obtained by the first measurement loop (2) in order to process, convert, transmit and/or display in real time the resulting signal (SR) representing the EGG, which is essentially devoid of noise.

10. A device according to claim 9 **characterized in that** all the modules (6, 6', 7, 8, 9, 10) are grouped within a shielded housing (12) forming a Faraday cage which is connected to the electrodes (3, 3'; 5, 5') by resistant, flexible connection wires (2", 4") and which is not in direct contact with the patient's skin.

**11.** A device according to either of claims 9 and 10 **characterized in that** the two acquisition modules (6, 6') comprise a common electrode (3', 5').

**12.** A device according to any one of claims 9 through 11 **characterized in that** the different elements and portions forming said device consist essentially of non-magnetic materials, making it completely compatible for use in a magnetically sensitive, turbulent environment.


**Patentansprüche**

**1.** Verfahren zur Erfassung des Elektrokardiogramms (EKG), im wesentlichen geräuschlos, eines Patienten, der sich in einer elektromagnetisch störenden Umgebung befindet, die ein Rauschen in dem Messsignal erzeugt, das darin besteht, an dem Patienten in Höhe seiner Herzregion ein erstes Messsignal (S1) zu ermitteln, das ein EKG-Signal mit Rausch Zugabe ist, als aufgenommenes Signal über zwei Elektroden (3, 3') das zu einer ersten Messschleife (2) gehört, und das darin besteht, außerdem und gleichzeitig am Patienten (1) durch eine zweite Messschleife (4), die sich von der ersten unterscheidet, mit zwei Elektroden (5, 5'), ein zweites Messsignal (S2) zu ermitteln, das mindestens das Rauschen enthält, dann in Echtzeit zu dem ersten EKG-Signal mit Rauschen (S1) das zweite Messsignal (S2) zu addieren oder davon zu subtrahieren, das mindestens das Rauschen enthält, je nach Polarität des Rauschens in letzterem Signal (S2) im Vergleich zu der des Rauschens in dem EKG-Signal mit Rauschen (S1), die zweite Messschleife (4) ist so angelegt, um allein die Aufnahme des EKG-Signals neben dem Rauschen oder vermischt mit dem Rauschen zu gestatten, wobei das EKG-Signal eine niedrigere Amplitude aufweist als das von der ersten Messschleife (2) gesammelte EKG-Signal und schließlich in Echtzeit das Endsignal (SR), das repräsentativ für das EKG und im wesentlichen rauschfrei ist, zu behandeln, umzuwandeln, zu übertragen und/oder anzuzeigen.

**2.** Verfahren zur Erfassung gemäß Anspruch 1, **gekennzeichnet dadurch, dass** die zweite Messschleife (4) derart angelegt ist, dass, wenn sie eine repräsentative Signalkomponente für das EKG neben oder vermischt mit dem Rauschsignal aufnimmt, besagte repräsentative Signalkomponente für das EKG eine entgegengesetzte Polarität und/oder eine Amplitude niedriger als die der repräsentativen Signalkomponente für das EKG aufweist, das von der ersten Messschleife (2) erfasst wurde.

**3.** Verfahren zur Erfassung gemäß Anspruch 1 oder 2, **gekennzeichnet dadurch, dass**, falls ein Patient (1) in einem Magnetresonanz-Bildgebungsgerät oder einem analogen Magnetresonanzanalysegerät liegt, die scheinbaren Ausmaße für den Magnetfluss, in einer Ebene (PP) senkrecht zu der Richtung des Hauptfeldes (Bo), der ersten und zweiten Messschleife (2 und 4), gebildet durch die Verbindungsdrähte (2' und 4') der jeweiligen Elektroden (3, 3' und 5, 5') und dem Gewebe (2", 4"), das sich zwischen den beiden Elektroden (3, 3'; 5, 5') derselben Messschleife (2, 4) erstreckt, ähnlich oder gar identisch sind.

**4.** Verfahren zur Erfassung gemäß einem beliebigen der Patentansprüche 1 oder 3, **gekennzeichnet dadurch, dass** die erste und zweite Messschleife (2 und 4) eine gemeinsame Elektrode (3', 5') enthalten.

**5.** Verfahren zur Erfassung gemäß einem beliebigen der Patentansprüche 1 bis 4, **gekennzeichnet dadurch, dass** die erste und zweite Messschleife (2 und 4) durch die Elektroden durchgeht und deutlich symmetrisch angeordnet ist im Verhältnis zur Mittelebene (PM) des Patienten (1) lotrecht zur coronaren Ebene (PC) von letzteren.

**6.** Verfahren zur Erfassung gemäß Anspruch 5, **gekennzeichnet dadurch, dass** die Elektroden (3, 3'; 5 5') verschiedene oder mit einer gemeinsamen Elektrode der beiden Messschleifen (2 und 4) in Reihe angeordnet sind.

**7.** Verfahren zur Erfassung gemäß einem beliebigen der Patentansprüche 1 bis 4, **gekennzeichnet dadurch, dass** die drei Elektroden (3, 3'; 5, 5'), die die beiden Messschleifen (2 und 4) bilden, in einer dreieckigen Ausgestaltung in Höhe der Herzregion angeordnet sind, so dass die zweite Messschleife (4) eine repräsentative Signalkomponente für das EKG entgegengesetzter Polarität und im wesentlichen gleicher Amplitude zu der repräsentativen Signalkomponente für das von der ersten Messschleife (2) ermittelte EKG feststellt.

**8.** Verfahren zur Erfassung gemäß einem beliebigen der Patentansprüche 1 bis 7, **gekennzeichnet dadurch, dass** das Signal (SR), das das Ergebnis der Addition oder der Subtraktion der Differentialsignale (S1 und S2) ist, die von den beiden Messschleifen (2 und 4) geliefert wurden, von einem Tiefpass-Filter (8) behandelt wird, dann zwecks Frequenzmodulation einer Trägerfrequenz genutzt und schließlich zu einem Apparat (11) übertragen wird, der mit einem Anzeigemittel für das gefilterte EKG-Signal (SR) ausgestattet ist, nötigenfalls nach Wandeln in ein optisches Signal.

9. Aufnahmevorrichtung für die Aufnahme in Echtzeit des EKG-Signals eines Patienten, der sich in einer elektromagnetisch störenden Umgebung befindet, insbesondere einer Magnetresonanzuntersuchung unterzogen wird, die mit einem Anzeigeapparat verbunden sein kann, und durch die die Durchführung des Verfahrens gemäß einem beliebigen der Patentansprüche 1 bis 9 möglich wird, wobei die Vorrichtung hauptsächlich einerseits aus zwei Modulen (6, 6') zur Entnahme von physiologischen Signalen (S1 und S2) besteht, deren erstes (S1) ein EKG-Signal mit Rauschen ist und das zweite (S2) ein Messsignal, das mindestens das Rauschen enthält, wobei die Module durch zwei verschiedene Messschleifen (2 und 4) gebildet sind, von denen jede ein Paar Elektroden (3, 3'; 5, 5') umfasst, die dazu ausgelegt sind, auf dem Patienten (1) plaziert zu werden, und andererseits aus einem Summier- (7) oder Differenzier-Modul für die Signale (S1 und S2), die von den beiden Entnahmemodulen (6, 6') geliefert werden, und schließlich aus Modulen (8, 9, 10) zur Behandlung, vor allem zum Filtern und Wandeln des Endsignals (SR), das von besagtem Summiermodul (7) oder Differenzierschaltung geliefert wird, wobei die zweite Messschleife (4) angelegt ist, um die Aufnahme des EKG-Signals zu gestatten, zusätzlich zu dem Rauschen oder vermischt damit, wobei das EKG-Signal eine niedrigere Amplitude als die des von der ersten Messschleife (2) gesammelten EKG-Signals aufweist, um das Endsignal (SR), das repräsentativ für das EKG und im wesentlichen ohne das (10) Rauschen ist, zu behandeln, umzuformen, zu übertragen und/oder in Echtzeit anzuzeigen.

10. Vorrichtung gemäß Anspruch 9, **gekennzeichnet dadurch, dass** alle Module (6, 6', 7, 8, 9, 10) in einem geschirmten Gehäuse (12) gesammelt sind, das einen Faraday-Käfig bildet, der mit den Elektroden 15 (3, 3'; 5, 5') durch flexible Widerstands-Verbindungsdrähte verbunden ist (2", 4") und nicht in direktem Kontakt mit der Haut des Patienten (1) steht.

11. Vorrichtung gemäß einem beliebigen der Patentansprüche 9 und 10, **gekennzeichnet dadurch, dass** die beiden Entnahmemodule (6, 6') eine gemeinsame Elektrode enthalten (3', 5').

12. Vorrichtung gemäß einem beliebigen der Patentansprüche 9 bis 11, **gekennzeichnet dadurch, dass** die verschiedenen Elemente und Teile, die die Vorrichtung bilden, im wesentlichen aus antimagnetische Materialien bestehen, was ihre Benutzung vollkommen Kompatibel mit einer sensiblen und störenden magnetischen Umgebung macht.

Y

Z

5  4  5′  3′  2  3  $B_o$

PC

4″  2″

1

X

PM

Fig.1

5  5′  3′  3

d  D  D  d

PM

Fig.2

5  3′,5′  3

D  D

PM

Fig.3

PM

$B_o$

5

3

3′,5′  PP

Fig.4

Fig. 5

Fig. 6A

Fig. 6B

avant séquence ←    Début séquence RMN ↓    pendant séquence →

Fig. 7A

avant séquence ←    Début séquence RMN ↓    pendant séquence →

Fig. 7B

EP 1 050 270 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2685968
- EP 0247991 A
- FR 2704131
- FR 2729071